# EUROPEAN PATENT SPECIFICATION

(11) **EP 2 030 580 B1**
(45) Date of publication and mention of the grant of the patent: **24.06.2015**
(21) Application number: 08015277.0
(22) Date of filing: 29.08.2008
(51) Int. Cl.: A61B 17/135, A61B 5/022

(54) **Blood pressure measurement cuff**
Manschette zur Blutdruckmessung
Brassard d'un tensiomètre pour mesurer la pression artérielle

(30) Priority: 30.08.2007 JP 2007223871; 19.10.2007 JP 2007272153
(43) Date of publication of application: 04.03.2009
(73) Proprietor: Nihon Kohden Corporation, Shinjuku-ku Tokyo (JP)
(72) Inventor: Todokoro, Noriaki, Tokyo (JP); Ooishi, Teishi, Tokyo (JP)
(74) Representative: Grünecker Patent- und Rechtsanwälte PartG mbB

(56) References cited:
- WO-A-2006/133539
- US-A- 5 411 518
- US-A- 5 660 182
- US-A1- 2001 005 777
- US-A1- 2004 186 385
- US-A1- 2005 015 015
- US-A1- 2005 182 331
- US-A1- 2006 184 054

## Description

The present invention relates to a blood pressure measurement cuff that measures blood pressure while being wrapped around an arm (a brachial portion) of a subject and inflated to apply pressure to the arm, and more particularly, effectively to a blood pressure measurement cuff suitable for long-term use.

A related-art blood pressure measurement cuff has a structure in which an air bag made from rubber is housed in an exterior bag formed from cloth that does not exhibit any stretching property. In the blood pressure measurement cuff, a sheet having a loop-implanted surface of a Hook-and-Loop fastener is provided to a portion of one side of the exterior bag, and a sheet having a hook-implanted surface of the Hook-and-Loop fastener is provided to a portion of the other side of the exterior bag. The cuff is wrapped around an arm and fixed by causing the loop to engage with the hook.

After the blood pressure measurement cuff is wrapped around the arm, air is injected by way of a tube joined to the air bag, to thus inflate the air bag and hinder blood flow. Subsequently, the air is deflated by way of the tube so as to reduce the cuff pressure gradually, thereby measuring blood pressure.

Wrinkles arise in the side of such a related-art blood pressure measurement cuff that is in contact with the arm when the cuff is wrapped around the arm. In addition to hardness of the cloth of the exterior bag, the wrinkles can pinch the skin of the arm when the air bag is inflated. In some cases, subcutaneous bleeding arises.

In light of the circumstance, the present inventors have already proposed a blood pressure measurement cuff that prevents occurrence of wrinkles, which cause a problem in an exterior bag, when used while being wrapped around a predetermined area of a living body; that prevents transmission of fine wrinkles to a skin surface of the living body; that securely prevents occurrence of subcutaneous bleeding, which would otherwise be caused by intensive local friction with the skin of the living body; and that can easily achieve appropriate close contact with the predetermined area of the living body (refer to JP-A-2008-99944).

The blood pressure measurement cuff of the proposal houses an air bag in the exterior bag and adopts a configuration in which a first slide sheet is movably interposed between an interior surface on an internal peripheral surface of the exterior bag that houses the air bag and an inner peripheral surface of the air bag and in which a second slide sheet is connected, at both longitudinal ends thereof, to the outside of the inner peripheral side surface of the exterior bag.

A proposed fluid bag (a sheet material forming the fluid bag has a thickness of 0.15 mm or less) is formed from a sheet material that is thinner than a hitherto-used sheet material, thereby reducing the influence of wrinkles arising in the inner peripheral sheet by shortening a difference between a circumferential length of the outer peripheral sheet and a circumferential length of the inner peripheral sheet while a hemodynamometer cuff is wrapped around the living body (see JP-A-2006-218178).

However, an effect for preventing occurrence of subcutaneous bleeding, which would otherwise be caused by the blood pressure measurement cuff, is limited, and in particular the effect cannot be said to be sufficient when the cuff is intermittently used over long term.

In the related-art blood pressure measurement cuff, the tube connected to the air bag is soft. Then, as a result of the tube being kinked during measurement or a subject, or the like, laying his/her body on the tube, problems arise failure in measurement or continually keep pressing the arm while the air bag remains inflated.

In contrast, there is another proposed blood pressure measurement cuff configured such that a tube, which is connected to a tourniquet and which is made from an elastic material, periodically changes in thickness along a single circumferential direction and that thicknesses of points symmetrical about an axis (see JP-A-2004-223102) become contradictory in terms of the increase and decrease in thickness. By means of the configuration, there is yielded an advantage of a tube being less susceptible to flexion during the course of being handled for measuring blood pressure when compared with the tube whose thickness is formed in such a way that positions located symmetrically about the axis become coincident in terms of the increase and decrease in thickness. Further, even when the tube is bent, a flow cross section for a pressured gas is ensured.

However, in the above configuration of the tube, a hole shape achieved along the cross section of the tube is not circular, and hence there is a problem which is difficult to connect the tube appropriately with a hitherto-used connector whose cross-section assumes a circular shape.

Further prior art is known from document US 5 660 182 which discloses a blood pressure measurement cuff comprising the features of the preamble of claims 1 and 12.

It is an object of the invention to provided a blood pressure measurement cuff capable of safely, appropriately performing measurement even when the cuff is intermittently used for long term.

In order to achieve the object, according to the invention, there is provided a blood pressure measurement cuff, **according to claim 1.**

The sheet may include at least one layer

The first surface may face to an arm of a subject when the cuff is wrapped around the arm of the subject

The sheet may be integrally provided on the first surface of the first bag.

An air passage may be formed between the sheet and the first bag.

The sheet may be integrally provided on the second inner surface of the second bag.

The blood pressure measurement cuff may further include a tube, connected to the first bag, through which air inflate or deflate the first bag.

The tube may include a wall portion, and a through hole enclosed by the wall portion and extending in a first direction. The wall portion may include a first portion and a second portion, the first portion being thicker than the second portion in a second direction perpendicular to the first direction.

The sheet include a loop-implanted surface or a hook-implanted surface of a Hook-and-Loop fastener.

The second bag may be formed from a thin cloth material, namely polyester, nylon or rayon.

A scale may be provided on the second bag.

According to the invention, there is also provided a blood pressure measurement cuff, according to claim 12.

### BRIEF DESCRIPTION OF THE DRAWINGS

Fig. 1 is a plane view showing a state in which an air bag and an exterior bag are separated in a first embodiment of a blood pressure measurement cuff related to the present invention.
Fig. 2 is a cross-sectional view showing a tube used in embodiments of the blood pressure measurement cuff related to the present invention.
Fig. 3A is a cross-sectional view along A-A line in Fig. 1.
Fig. 3B is a cross-sectional view in which the arrangement of a sheet is changed from that of Fig. 3A.
Fig. 4 is a plane view showing a back surface of the exterior bag in the blood pressure measurement cuff related to the present invention shown in Fig. 1.
Fig. 5 is a plane view showing a state in which the air bag is housed in the exterior bag in the first embodiment of the blood pressure measurement cuff related to the present invention.
Fig. 6 is a cross-sectional view along B-B line in Fig. 5.
Fig. 7 is a plane view showing a state in which an air bag is withdrawn from an exterior bag in a second embodiment of the blood pressure measurement cuff related to the present invention.
Fig. 8 is a cross-sectional view along C-C line in Fig. 7 showing a state in which the air bag is housed in the exterior bag.
Fig. 9 is a plane view showing another example of the second embodiment of the blood pressure measurement cuff related to the present invention.
Fig. 10 is a plane view showing an example of a design pattern on a surface of the blood pressure measurement cuff related to the present invention.

### DETAILED DESCRIPTION OF PREFERRED EMBODIMENTS

Embodiments of a blood pressure measurement cuff of the present invention will be described hereunder by reference to the accompanying drawings. In the drawings, the same reference numerals are provided to the same constituent elements, and their repeated explanations are omitted.

### <First Embodiment>

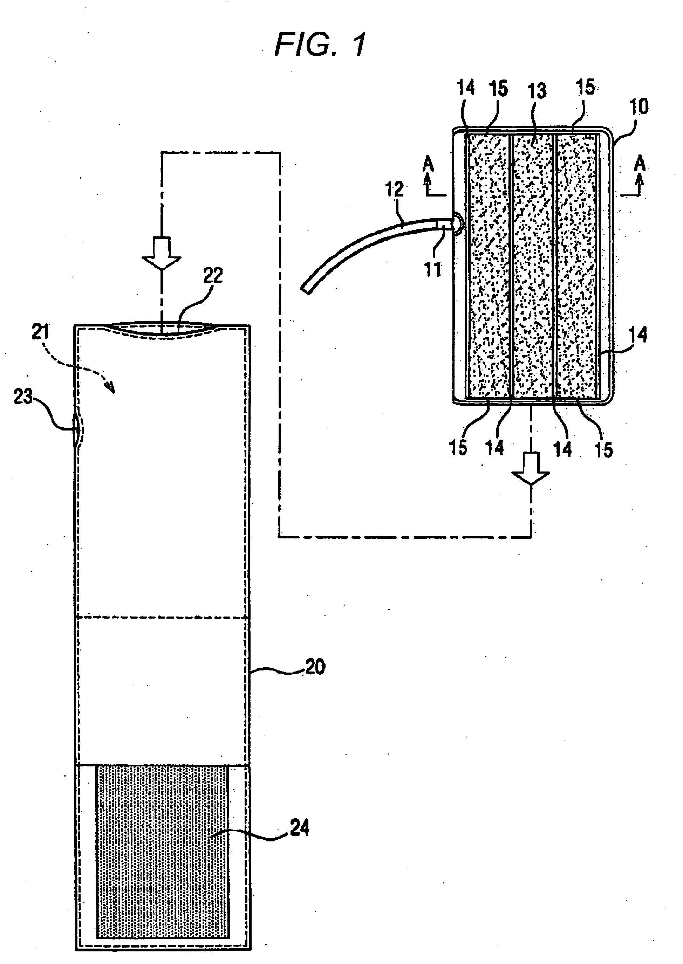

As shown in Fig. 1, a blood pressure measurement cuff of the first embodiment includes an air bag 10 and an exterior bag 20. The air bag 10 is formed from an elastic material, such as rubber, and a connector 11 is attached to a side portion of the air bag 10, and the connector 11 is inserted and joined to a tube 12. A sheet 13 is provided to one surface of the air bag 10, and the air bag 10 and the sheet 13 are integrated into a single body.

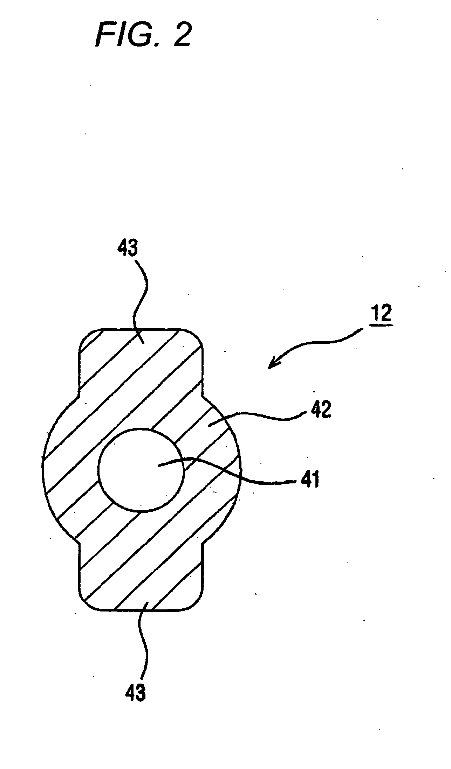

The tube 12 joined to the connector 11 assumes a cross-sectional profile, as shown in Fig. 2, in which exterior wall portions opposing each other along a diameter of a cylinder are made thick, and the tube has a structure in which an interior passage 41 is not clogged even in a state where the tube is kinked. Specifically, the tube has the interior passage 41 formed from a circular hole opened in the cross-sectional center of the tube 12; a tube main body 42 that concentrically encloses the interior passage 41 and whose cross section is sandwiched between two circles; and thick portions 43 that are formed at positions opposing each other along the diameter of the tube main body 42 and that serve as thick exterior wall portions protruding outside of the tube main body 42.

The sheet 13 include at least one layer and is made from a material having a characteristic that a shallow curved shape is maintained with no wrinkle therein when wrapped around an arm of a subject, and that no wrinkle is remained and a bending tendency is hard to occur even after bent. A loop-implanted surface or a hook-implanted surface of a Hook-and-Loop fastener, such as a Velcro Tape (Registered Trademark), can be mentioned as such a material. In case that a Hook-and-Loop fastener is applied to the present invention, not only a Hook-and-Loop fastener having both of a hook-implanted face and a loop-implanted face, but also a Hook-and-Loop fastener having one of a hook-implanted face and a loop-implanted face may be used as the sheet 13. In fact, the loop-implanted surface of the Hook-and-Loop fastener is selected as the material of the sheet 13.

The thickness of the sheet 13 preferably ranges from 0.5 mm to 3 mm. Specifically, when the thickness of the sheet 13 is 0.4 mm or less, occurrence of wrinkles is observed though they are still tiny. When the thickness exceeded 3 mm; namely, 3.5 mm or more, there arose a case where a pulse wave could not be appropriately detected. Accordingly, adopting a thickness within a range of 0.5 mm to 3 mm is desirable.

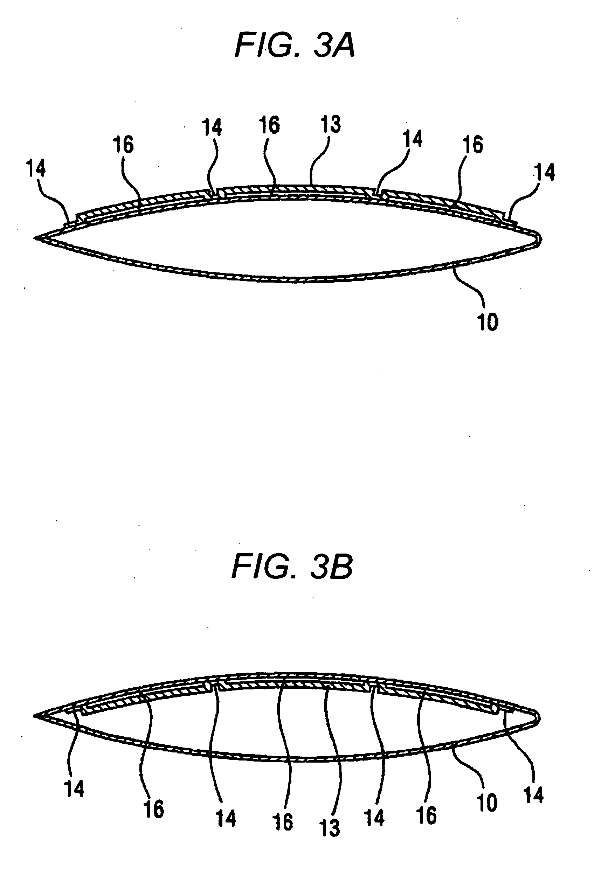

An adhesive of; for instance, thermal adhesion type, is applied over a back surface of the sheet 13. As shown in Figs. 1 and 3A, the adhesive is longitudinally fused and fixed in the form of four stripes, whereby four stripe-shaped adhesive sections 14 are formed. Longitudinal ends of the sheet 13 remains unfused and opened, thereby forming air vent holes 15 communicating with the outside air. An air passage 16 remaining in communication with the air vent holes 15 is constituted between the sheet 13 and the air bag 10. Therefore, in a situation where the cuff is used particularly in a tropical area or a subtropical area, even when an air existing between the sheet 13 and the air bag 10 is inflated, the air escapes from the air passage 16 via the air vent holes 15. Hence, a problem that the sheet 13 is exfoliated from the air bag 10, which would otherwise be cause by inflation of an air when edges of the sheet 13 are fixedly fused, can be prevented. As shown in Fig. 3B, the sheet 13 may also be thermally fused and fixed in the air bag 10.

The exterior bag 20 has a length that is about twice the longitudinal length of the air bag 10 and is formed from a thin cloth material having a characteristic that no wrinkle occurs therein when wrapped around the arm and that no wrinkle is remained and a bending tendency is hard to occur even after bent. Polyester, nylon, rayon, and the like, can be mentioned as the cloth material.

The exterior bag 20 is formed from two clothes that are superimposed with each other and that are made of the above material; and has a housing chamber 21 for housing the air bag 10. A large opening 22 used for inserting and withdrawing the air bag 10 into and from the housing chamber 21 is formed at one longitudinal end of the exterior bag 20. A small opening 23 that allows protrusion of the tube 12 to the outside is formed at a position on the exterior bag 20 where the connector 11 to be connected to the tube 12 is situated while the air bag 10 is housed in the housing chamber 21.

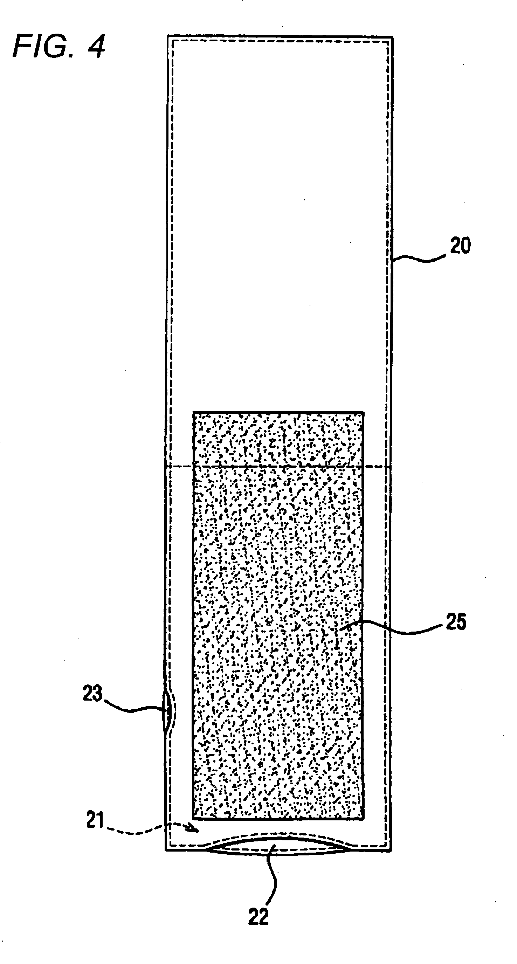

A hook-implanted surface 24 of the Hook-and-Loop fastener whose length is about quarter of the longitudinal length of the exterior bag 20 is provided to a surface of the exterior bag 20 which is opposite to the housing chamber 21 with respect to a center line of the exterior bag 20, sandwiched between the hook-implanted surface 24 and the housing chamber 21, and which comes inside when the blood pressure measurement cuff is wrapped around the arm. Further, a loop-implanted surface 25 of the Hook-and-Loop fastener is provided to a back surface of the exterior bag 20 which substantially corresponds to the housing chamber 21 and which is disposed at the opposite side to the surface to which the hook-implanted surface 24 of the Hook-and-Loop fastener is provided (see Fig. 4).

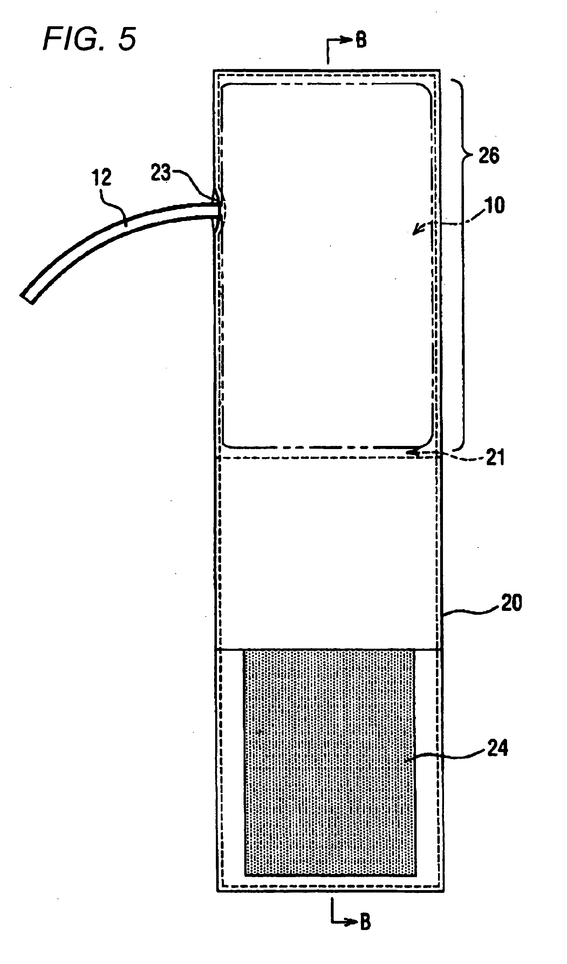

The air bag 10 is folded and inserted into the housing chamber 21 of the exterior bag 20 configured as mentioned above by way of the large opening 22 as indicated by an arrow in Fig. 1, and the tube 12 is caused to protrude outside from the small opening 23. The folded air bag 10 is spread in the housing chamber 21, and the large opening 22 is seamed to close, thereby completing a blood pressure measurement cuff, such as that shown in Fig. 5.

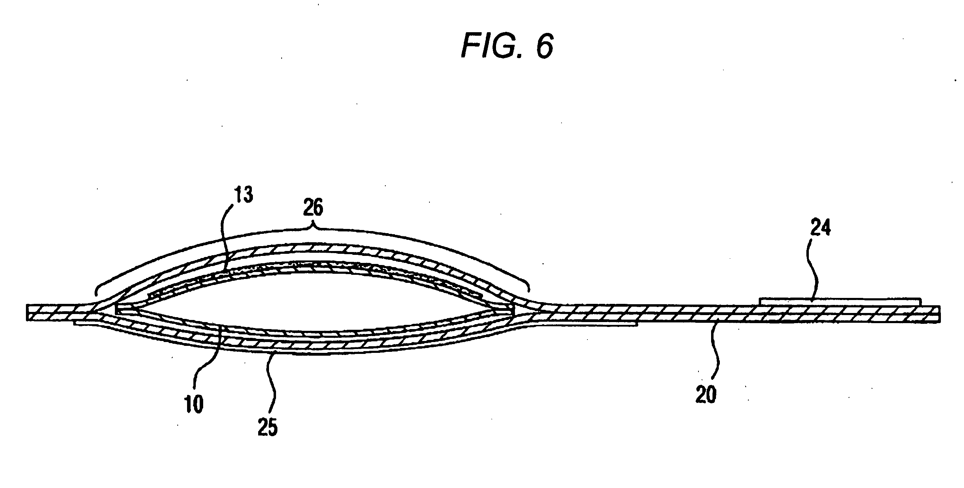

As shown in Fig. 6, the sheet 13 adhered to the air bag 10 faces to a portion of the exterior bag 20 to which the hook-implanted surface 24 is provided and which is a wrap section 26 that faces to the arm when the cuff is wrapped around the arm. When the cuff is wrapped around the arm, the sheet 13 is formed in a shallow curved shape with no wrinkle therein. Even when the air bag 10 is inflated, the shallow curved shape is maintained. Hence, the wrap section 26 that is in contact with the sheet 13 maintains a circular cross-sectional profile, and no wrinkle occurs therein when the cuff is wrapped around the arm, so that a cause of subcutaneous bleeding does not arise.

Even when any portion of the air bag 10 is bent for the purpose of housing into the exterior bag 20, or the like, since the sheet 13 is formed from a material having a characteristic that no wrinkle is remained and a bending tendency is hard to occur even after bent, the sheet 13 is formed in a shallow curved shape with no wrinkle therein, even when the cuff is already wrapped around the arm at the time of remeasurement. Moreover, since the wrap section 26 of the exterior bag 20 is also formed from a thin cloth material having a characteristic that no wrinkle is remained and a bending tendency is hard to occur even after bent, a cause of subcutaneous bleeding does not arise.

The cross section of the tube 12 is as shown in Fig. 2, and the tube 12 becomes hard to bend in a direction interconnecting the thick portions 43, thereby preventing kinking of the interior passage 41 that is a through hole. Therefore, even when the tube 12 is bent as a result of the subject moving the arm in the case of long term, the tube is not kinked. Even when the subject is laid on the tube 12, the interior passage 41 is not closed, and hence measurement of blood pressure can be stably, appropriately, and continually carried out. In the present embodiment, the thick portions 43 are provided at two positions that are connected along the diameter of the cross section of the tube 12. No limitations are imposed on the number and shape of the thick portions, and the essential requirement for the thick portions is that portions of the tube main body are made thick, to prevent the interior passage 41 from kinking.

### <Second Embodiment>

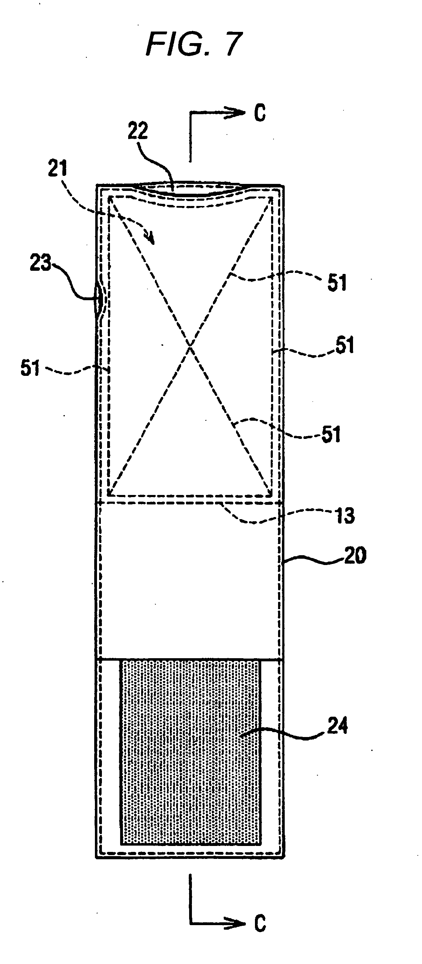

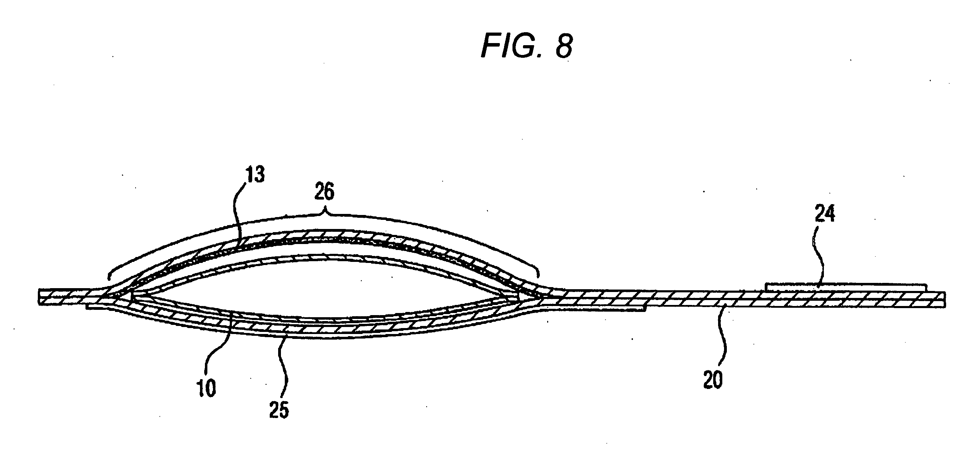

In a blood pressure measurement cuff of a second embodiment of the present invention, the sheet 13 is fixedly provided, as in the first embodiment, between the surface of the air bag 10, which is disposed on the arm side when the cuff is wrapped around the arm, and the surface of the exterior bag 20 which faces to the surface of the air bag 10 as shown in Figs. 7 and 8.

The sheet 13 is seamed and integrally fixed to the surface of the exterior bag 20 which faces to the arm-side surface of the air bag 10, when the cuff is wrapped around the arm. The sheet 13 is the Hook-and-Loop fastener having the hook-implanted surface and loop-implanted surface. Here, the orientation of the hook-implanted surface and the orientation of the loop-implanted surface in the sheet 13 are not particularly specified. Specifically, no problem arises even when either the hook-implanted surface or the loop-implanted surface of the sheet 13 faces to the arm-side surface of the air bag 10.

Reference numeral 51 denotes a seam resultant from seaming the sheet 13 to the exterior bag 20. In the present embodiment, seams are provided along edges of a square shape of the sheet 13 and also along diagonal lines of the square shape of the sheet 13. The manner of seaming is not limited to an example shown in Fig. 7, so long as the shallow curved shape is maintained with no wrinkle in the sheet 13 and the wrap section 26 that is in contact with the sheet 13 when the cuff is wrapped around the arm, and no wrinkle remains in the sheet 13 and the wrap section 26 and a bending tendency is hard to occur even after the sheet 13 and the wrap section 26 are bent, for instance, the sheet 13 may be integrated with the exterior bag 20 by providing several seams vertically and horizontally, not along the diagonal lines of the square shape of the sheet 13.

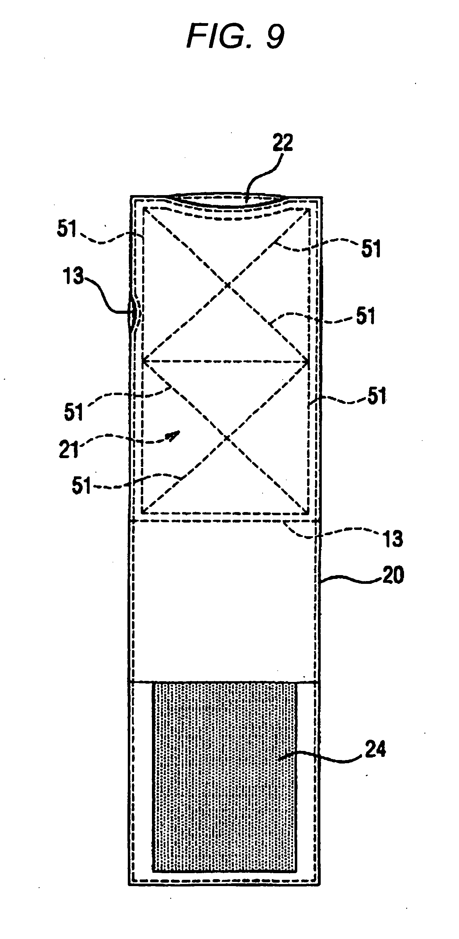

For instance, Fig. 9 shows another manner of seaming. In Fig. 9, seaming is performed along the edges of the square shape of the sheet 13. Also, seaming is performed in such a way that long sides of the square shape of the sheet 13 are divided into two equal parts, whereby two squares are produced. Further, seeming is performed along diagonal lines of the respective two squares. Thus, a seam 51 shown in Fig. 9 is produced. A technique of fixing the sheet 13 is not always limited to seaming, and bonding, or the like, can also be adopted.

The configuration of the blood pressure measurement cuff of the second embodiment is identical with that of the blood pressure measurement cuff of the first embodiment except the above mentioned points. The blood pressure measurement cuff of the second embodiment can be used as shown in Fig. 5 while the air bag 10 is inserted into and housed in the housing chamber 21 of the exterior bag 20 to which the sheet 13 is fixed. Even in this case, as in the case of the blood pressure measurement cuff of the first embodiment, there can be yielded an advantage of: the sheet 13 and the wrap section 26 being in contact with the sheet 13 which maintain a circular cross-sectional profile; no wrinkle occurring when the cuff is wrapped around the arm; and a cause of subcutaneous bleeding being prevented.

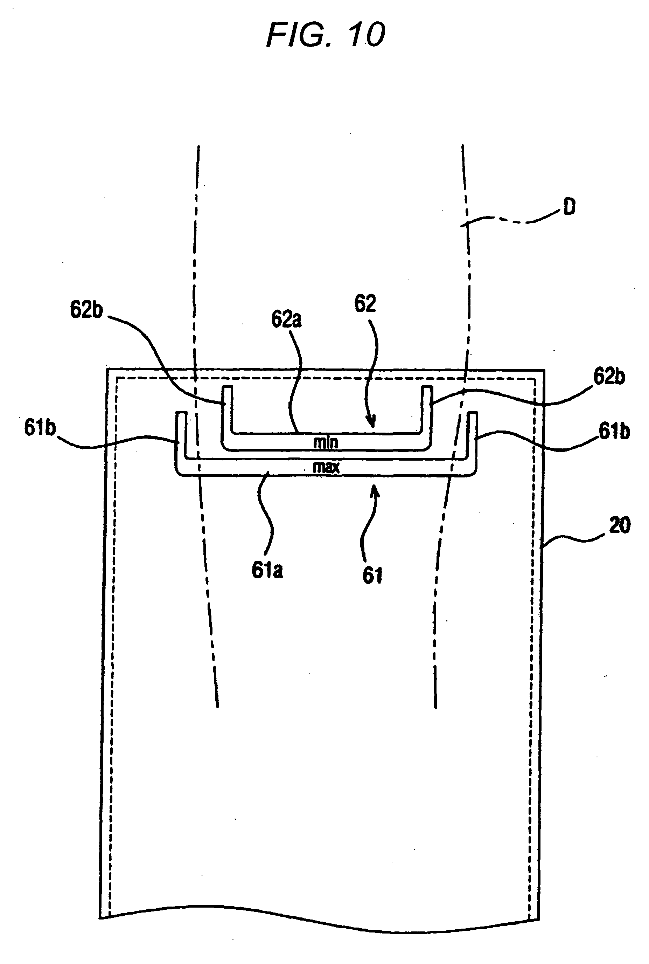

In the blood pressure measurement cuffs of the first and second embodiments, scale indicators 61 and 62, such as those shown in Fig. 10, are provided at, for instance, an end portion of the surface of the exterior bag 20 where the loop-implanted surface 25 is not provided in the surface of the exterior bag 20 to which the loop-implanted surface 25 is provided.

The scale indicators 61 and 62 can be printed by means of inkjet printing, or the like. Print colors of the scale indicators 61 and 62 can be appropriately adopted in accordance with a color of the exterior bag 20. The scale indicators 61 and 62 are marks showing the size of the arm of the user for which the cuff is suitably used. The respective scale indicators 61 and 62 have width bars 61a and 62a conforming to the width of a part of a subject, for instance the arm, as well as having side bars 61b and 62b that are provided at both ends of the respective width bars 61a and 62a and that show directions along the side of the part of the subject.

When the blood pressure measurement cuff is used, an arm D is laid with respect to the scale indicators 61 and 62 as shown in Fig. 10 in such a way that, for instance, an elbow-side of the cuff protrudes from the exterior bag 20 and that a fingertip-side of the cuff comes to the center of the exterior bag 20. At this time, as shown in Fig. 10, the blood pressure measurement cuff is expressed as being suitable for use when the arm D is located inside the side bars 61b of the scale indicator 61 and when the scale indicator 62 is hidden behind the arm D.

Specifically, the scale indicator 61 shows the maximum size of an appropriate arm, and the scale indicator 62 shows the minimum size of the appropriate arm. When the scale indicators 61 and 62 show that the blood pressure measurement cuff is not appropriate, another separately-prepared blood pressure measurement cuff that has a different length and a different width and that is appropriate for another user can be used, and appropriate measurement of blood pressure can be performed.

Although the preferred embodiments of the present invention have been described thus far, the present invention is not limited to the embodiments. The present invention is susceptible to many design changes within the scope of the claims.

According to an aspect of the present invention, a sheet fixedly interposed between a surface of an air bag which faces to an arm when a blood pressure measurement cuff is wrapped around the arm and a surface of an exterior bag which faces to the surface of the air bag, includes at least one layer. And, the sheet is formed from a material having a characteristic that a shallow curved shape is maintained with no wrinkle when wrapped around the arm and no wrinkle is remained and a bending tendency is hard to occur even after bent. Hence, even when the cuff is intermittently used for long term, a skin of the arm is not nipped between wrinkles as a result of occurrence of wrinkles, and measurement can be performed safely, appropriately while preventing occurrence of subcutaneous bleeding.

According to an aspect of the present invention, the sheet provided integrally on the surface of the air bag which faces to the arm when the cuff is wrapped around the arm is configured as mentioned previously. Hence, even when the cuff is intermittently used for long term, a skin of the arm is not nipped between wrinkles as a result of occurrence of wrinkles, and measurement can be performed safely, appropriately while preventing occurrence of subcutaneous bleeding.

According to an aspect of the present invention, the sheet provided integrally on the surface of the exterior bag which faces to the arm-side surface of the air bag when the cuff is wrapped around the arm is configured as mentioned previously. Hence, even when the cuff is intermittently used for long term, a skin of the arm is not nipped between wrinkles as a result of occurrence of wrinkles, and measurement can be performed safely, appropriately while preventing occurrence of subcutaneous bleeding.

According to an aspect of the present invention, an air passage in communication with air vent holes is formed between the sheet and the air bag. Hence, occurrence of a problem that the sheet is exfoliated from the air bag when an air existing between the sheet and the air bag is inflated, can be prevented.

According to an aspect of the present invention, the tube is structured so as to have an interior passage formed from a center circular hole when viewed in the cross-sectional profile of the tube; a tube main body that concentrically spreads outside with respect to the interior passage when viewed in the cross-sectional profile of the tube; and thick portions that bulge outside from the tube main body and that prevent the interior passage from kinking when the tube main body is bent. Hence, the opportunity of kinking of the interior passage is eliminated, and measurement can be safely, appropriately performed even when the cuff is used for long term.

According to an aspect of the present invention, the exterior bag is formed from a thin cloth material having a characteristic that no wrinkle occurs when wrapped around the arm and that no wrinkle is remained and a bending tendency is hard to occur even after bent. Accordingly, even when the cuff is intermittently used for long term, a skin of the arm is not nipped between wrinkles as a result of occurrence of wrinkles in the exterior bag, and measurement can be performed safely, appropriately while preventing occurrence of subcutaneous bleeding.

According to an aspect of the present invention, scale indicators showing the size of an arm of a user for which use of the cuff is appropriate are provided on the exterior bag. Hence, there is an advantage of the ability to easily determine whether use of the cuff is appropriate or not by applying the scale indicators to the arm of a patient, or the like, who uses the cuff, and comparing the arm with the scale indicators and to ensure appropriate measurement.

## Claims

1. A blood pressure measurement cuff, comprising:
a first bag (10), including a first surface;
a second bag (20) configured to be wrapped around an arm of a subject and housing the first bag (10) and including a second inner surface facing to the first surface in an unwrapped condition; and
a sheet (13) fixed to one of the first surface and the second inner surface,
**characterized in that**
the sheet is capable of being bent with no wrinkles therein, and
includes a loop-implanted surface or a hook-implanted surface of a Hook-and-Loop fastener.

2. The blood pressure measurement cuff according to claim 1, wherein the sheet (13) includes at least one layer.

3. The blood pressure measurement cuff according to claim 1, wherein the first surface faces to an arm of a patient when the cuff is wrapped around the arm.

4. The blood pressure measurement cuff according to claim 3, wherein the sheet (13) is integrally provided on the first surface of the first bag (10).

5. The blood pressure measurement cuff according to claim 4, wherein an air passage (16) is formed between the sheet (13) and the first bag (10).

6. The blood pressure measurement cuff according to claim 3, wherein the sheet (13) is integrally provided on the second inner surface of the second bag (20).

7. The blood pressure measurement cuff according to claim 1, further comprising: a tube (12), connected to the first bag (10), through which air inflate or deflate the first bag (10).

8. The blood pressure measurement cuff according to claim 7, wherein the tube (12) includes a wall portion, and a through hole (41) enclosed by the wall portion and extending in a first direction, and
the wall portion includes a first portion (43) and a second portion (42), the first portion (43) being thicker than the second portion (42) in a second direction perpendicular to the first direction.

9. The blood pressure measurement cuff according to claim 1, wherein the second bag (20) is formed from a thin cloth material, namely polyester, nylon or rayon.

10. The blood pressure measurement cuff according to claim 1, wherein a scale (61, 62) is provided on the second bag (20).

11. The blood pressure measurement cuff according to claim 1, wherein the sheet (13) has a thickness within a range of 0.5 mm to 3 mm.

12. A blood pressure measurement cuff, comprising
a first bag (10), including an inside surface and an outside surface;
a second bag (20), housing the first bag (10),
**characterized in that**
a sheet (13) is fixed to the inside surface,
and the sheet (13) includes a loop-implanted surface or a hook-implanted surface of a Hook-and-Loop fastener.

## Patentansprüche

1. Eine Blutdruckmessmanschette, aufweisend:
einen ersten Beutel (10), inklusive einer ersten Oberfläche,
einen zweiten Beutel (20), der konfiguriert ist, um einen Arm eines Subjekts gewickelt zu werden, und der den ersten Beutel (10) beinhaltet und eine zweite innere Oberfläche aufweist, die gegenüber der ersten Oberfläche in einem unumwickelten Zustand liegt, und
eine Bahn (13), die entweder an der ersten Oberfläche oder an der zweiten inneren Oberfläche fixiert ist, **dadurch gekennzeichnet, dass**
die Bahn (13) in der Lage ist gebogen zu werden ohne Falten darin, und eine schleifenimplantierte Oberfläche oder eine hakenimplatierte Oberfläche eines Haken- und Schleifenbefestigers beinhaltet.

2. Die Blutdruckmessmanschette gemäß Anspruch 1, wobei die Bahn (13) zumindest eine Schicht beinhaltet.

3. Die Blutdruckmessmanschette gemäß Anspruch 1, wobei die erste Oberfläche gegenüber einem Arm eines Patienten liegt, wenn die Manschette um den Arm gewickelt ist.

4. Die Blutdruckmessmanschette gemäß Anspruch 3, wobei die Bahn (13) integrativ an der ersten Oberfläche des ersten Beutels (10) vorgesehen ist.

5. Die Blutdruckmessmanschette gemäß Anspruch 4, wobei eine Luftpassage (16) zwischen der Bahn (13) und dem ersten Beutel (10) geformt ist.

6. Die Blutdruckmessmanschette gemäß Anspruch 3, wobei die Bahn (13) integrativ an der zweiten inneren Oberfläche des zweiten Beutels (20) bereitgestellt ist.

7. Die Blutdruckmanschette gemäß Anspruch 1, weiter aufweisend:
einen Schlauch (12), der mit dem ersten Beutel (10) verbunden ist, durch den Luft strömt, um den ersten Beutel (10) aufzublasen oder abzublasen.

8. Die Blutdruckmessmanschette gemäß Anspruch 7, wobei der Schlauch (12) einen Wandabschnitt beinhaltet und ein Durchgangsloch 41, das von dem Wandabschnitt umschlossen ist und sich in einer ersten Richtung erstreckt, und der Wandabschnitt einen ersten Abschnitt (43) und einen zweiten Abschnitt (42) beinhaltet und der erste Abschnitt (43) dicker ist als der zweite Abschnitt (42) in einer zweiten Richtung rechtwinklig zu der ersten Richtung.

9. Die Blutdruckmessmanschette gemäß Anspruch 1, wobei der zweite Beutel (20) von einem dünnen Stoffmaterial geformt ist, wie z. B. Polyester, Nylon oder Kunstseide.

10. Die Blutdruckmessmanschette gemäß Anspruch 1, wobei eine Skale (61, 62) an dem zweiten Beutel (20) vorgesehen ist.

11. Die Blutdruckmessmanschette gemäß Anspruch 1, wobei die Bahn (13) eine Dicke in einem Bereich von 0,5 mm bis 3 mm hat.

12. Eine Blutdruckmessmanschette, aufweisend:
einen ersten Beutel (10) inklusive einer inneren Oberfläche und einer äußeren Oberfläche,
einen zweiten Beutel (20), der den ersten Beutel (10) beherbergt,
**dadurch gekennzeichnet, dass**
eine Bahn (13) an der inneren Oberfläche fixiert ist,
und die Bahn (13) eine schleifenimplantierte Oberfläche oder eine hakenimplantierte Oberfläche eines Haken- und Schleifenbefestigers beinhaltet.

## Revendications

1. Manchette de mesure pour mesurer la pression artérielle comprenant :
un premier sac (10) incluant une première surface ;
un second sac (20) configuré pour être enroulé autour du bras d'un patient et contenant le premier sac (10), et incluant une seconde surface intérieure tournée vers la première surface dans l'état non enroulé ; et
une feuille (13) fixée sur une surface parmi la première surface et la seconde surface, **caractérisée en ce que**
la feuille est capable d'être pliée sans ride dans celle-ci, et
comporte une surface dans laquelle sont implantées des boucles ou une surface dans laquelle sont implantés des crochets d'une fermeture autoagrippante.

2. Manchette de mesure pour mesurer la pression artérielle selon la revendication 1, dans laquelle la feuille (13) comporte au moins une couche.

3. Manchette de mesure pour mesurer la pression artérielle selon la revendication 1, dans laquelle la première surface est tournée vers le bras d'un patient lorsque la manchette de mesure est enroulée autour du bras.

4. Manchette de mesure pour mesurer la pression artérielle selon la revendication 3, dans laquelle la feuille (13) est prévue de manière intégrée sur la première surface du premier sac (10).

5. Manchette de mesure pour mesurer la pression artérielle selon la revendication 4, dans laquelle un passage d'air (16) est formé entre la feuille (13) et le premier sac (10).

6. Manchette de mesure pour mesurer la pression artérielle selon la revendication 3, dans laquelle la feuille (13) est prévue de manière intégrée sur la seconde surface intérieure du second sac (20).

7. Manchette de mesure pour mesurer la pression artérielle selon la revendication 1, comprenant en outre un tube (12) relié au premier sac (10) à travers lequel de l'air gonfle ou dégonfle le premier sac (10).

8. Manchette de mesure pour mesurer la pression artérielle selon la revendication 7, dans laquelle le tube (12) comporte une partie de paroi et un trou traversant (41) entouré par la partie de paroi et s'étendant dans une première direction, et
la partie de paroi comporte une première partie (43) et une seconde partie (42), la première partie (43) étant plus épaisse que la seconde partie (42) dans une seconde direction perpendiculaire à la première direction.

9. Manchette de mesure pour mesurer la pression artérielle selon la revendication 1, dans laquelle le second sac (20) est formé d'un matériau en tissu mince, à savoir, du polyester, du nylon ou de la rayonne.

10. Manchette de mesure pour mesurer la pression artérielle selon la revendication 1, dans laquelle une échelle (61, 62) est prévue sur le second sac (20).

11. Manchette de mesure pour mesurer la pression artérielle selon la revendication 1, dans laquelle la feuille (13) possède une épaisseur se situant dans la plage allant de 0,5 mm à 3 mm.

12. Manchette de mesure pour mesurer la pression artérielle comprenant :
un premier sac (10) incluant une surface intérieure et une surface extérieure ;
un second sac (20) contenant le premier sac (10),
**caractérisée en ce que**
une feuille (13) est fixée sur la surface intérieure,
et la feuille (13) comporte une surface dans laquelle sont implantées des boucles ou une surface dans laquelle sont implantés des crochets d'une fermeture autoagrippante.
